# EUROPEAN PATENT APPLICATION

(11) **EP 2 083 366 A2**
(43) Date of publication of application: **29.07.2009**
(21) Application number: 09250228.5
(22) Date of filing: 28.01.2009
(51) Int. Cl.: G06F 19/00

(54) **Methods and devices for predicting performance of a gastric restriction system**

(30) Priority: 28.01.2008 US 20850
(71) Applicant: Ethicon Endo-Surgery, Inc., Cincinnati, OH 45242 (US)
(72) Inventor: Ortiz, Mark S., Milford, Ohio 45150 (US); Dlugos, Daniel F., Jr., Middletown, Ohio 45044 (US); Marcotte, Amy L., Mason, Ohio 45040 (US); Plescia, David N., Cincinnati, Ohio 45227 (US)
(74) Representative: Tunstall, Christopher Stephen

(57) **Abstract**

Methods and devices are provided for predicting performance of a restriction system for a patient. In general, the methods and devices can allow detection and prediction of a trajectory of a particular patient attribute, such as weight loss. Using previously gathered data values, data values defining a future outcome can be predicted and compared with a desired future outcome. If the future outcome deviates from the desired future outcome, one or more corrective actions can be suggested to a patient and/or a health care provider to help align the patient's treatment plan with the desired future outcome rather than the currently predicted future outcome.

## Description

### FIELD OF THE INVENTION

The present invention relates to devices and methods for predicting performance of a gastric restriction system.

### BACKGROUND OF THE INVENTION

Obesity is becoming a growing concern, particularly in the United States, as the number of obese people continues to increase and more is learned about the negative health effects of obesity. Morbid obesity, in which a person is 100 pounds or more over ideal body weight, in particular poses significant risks for severe health problems. Accordingly, a great deal of attention is being focused on treating obese patients. One method of treating morbid obesity has been to place a restriction device, such as an elongated band, about the upper portion of the stomach. Gastric bands have typically comprised a fluid-filled elastomeric balloon with fixed endpoints that encircles the stomach just inferior to the esophageal-gastric junction to form a small gastric pouch above the band and a reduced stoma opening in the stomach. When fluid is infused into the balloon, the band expands against the stomach creating a food intake restriction or stoma in the stomach. To decrease this restriction, fluid is removed from the band. The effect of the band is to reduce the available stomach volume and thus the amount of food that can be consumed before becoming "full."

Food restriction devices have also comprised mechanically adjusted bands that similarly encircle the upper portion of the stomach. These bands include any number of resilient materials or gearing devices, as well as drive members, for adjusting the bands. Additionally, gastric bands have been developed that include both hydraulic and mechanical drive elements. An example of such an adjustable gastric band is disclosed in U.S. Pat. No. 6,067,991, entitled "Mechanical Food Intake Restriction Device" which issued on May 30, 2000, and is incorporated herein by reference. It is also known to restrict the available food volume in the stomach cavity by implanting an inflatable elastomeric balloon within the stomach cavity itself. The balloon is filled with a fluid to expand against the stomach walls and, thereby, decrease the available food volume within the stomach.

With each of the above-described food restriction devices, safe, effective treatment requires that the device be regularly monitored and adjusted to vary the degree of restriction applied to the stomach. With banding devices, the gastric pouch above the band will substantially increase in size following the initial implantation. Accordingly, the stoma opening in the stomach must initially be made large enough to enable the patient to receive adequate nutrition while the stomach adapts to the banding device. As the gastric pouch increases in size, the band may be adjusted to vary the stoma size. In addition, it is desirable to vary the stoma size in order to accommodate changes in the patient's body or treatment regime, or in a more urgent case, to relieve an obstruction or severe esophageal dilatation. Traditionally, adjusting a hydraulic gastric band required a scheduled clinician visit during which a Huber needle and syringe were used to penetrate the patient's skin and add or remove fluid from the balloon via an injection port. More recently, implantable pumps have been developed which enable non-invasive adjustments of the band. An external programmer communicates with the implanted pump using telemetry to control the pump. During a scheduled visit, a physician places a hand-held portion of the programmer near the gastric implant and transmits power and command signals to the implant. The implant in turn adjusts the fluid levels in the band and transmits a response command to the programmer.

During these gastric band adjustments, it has been difficult to determine how the adjustment is proceeding and whether the adjustment will have the intended effect. In an attempt to determine the efficacy of an adjustment, some physicians have utilized fluoroscopy with a Barium swallow as the adjustment is being performed. However, fluoroscopy is both expensive and undesirable due to the radiation doses incurred by both the physician and patient. Other physicians have instructed the patient to drink a glass of water during or after the adjustment to determine whether the water can pass through the adjusted stoma. This method, however, only assures that the patient is not obstructing and does not provide any information about the efficacy of the adjustment. Oftentimes, a physician may simply adopt a "try as you go" method based upon their prior experience, and the results of an adjustment may not be discovered until hours or days later, when the patient experiences a complete obstruction to the stomach cavity, or the band induces erosion of the stomach tissue due to excessive interface pressures against the band.

Accordingly, methods and devices are provided for use with an implantable restriction device, and in particular for diagnosing performance of an implantable restriction system.

### SUMMARY OF THE INVENTION

The present invention generally provides methods and devices for predicting performance of a gastric restriction system. In one embodiment, a method of affecting a weight loss treatment is provided that includes extrapolating a sequence of future data values using at least two data values gathered in relation to a patient having an implanted restriction device that can form a restriction in the patient. Extrapolating a sequence of future data values can include using, for example, any one of a linear extrapolation technique, a conic extrapolation technique, and a polynomial extrapolation technique. The gathered data values can be gathered using a device external to the patient and/or implanted and in communication with the restriction device. Furthermore, the gathered data values can reflect a patient condition including any one of weight, weight loss, weight gain, percent excess weight loss, body mass index, satiety level, body dimensions, heart rate, blood pressure, and breathing rate. In some embodiments, the gathered data values define a current trend, the desired sequence of future data values define a desired trend, and extrapolating a sequence of future data values includes extrapolating a remedial trend to align the current trend with the desired trend. In some embodiments, extrapolating a sequence of future data values includes extrapolating a sequence of future data values following the gathered data values.

The method also includes, if the sequence of future data values deviates from a desired sequence of future data values, determining a suggested corrective action to address the deviation. The suggested corrective action can include modifying a treatment plan of the patient, adjusting an amount of fluid disposed within the restriction device, advising the patient to alter a level of physical activity, recommending that the patient adjust diet, and/or recommending that the patient adjust eating habits. In some embodiments, the method also includes displaying a notice of the suggested corrective action on a display device.

In another embodiment, a method of affecting a weight loss treatment includes generating a plot showing a patient indicator plotted over time. The plot includes patient indicator data points related to past treatment history of a patient having an implantable restriction device that can form a restriction in the patient. The patient indicator data points can reflect a patient condition including any one of weight, weight loss, body mass index, satiety level, body dimension, heart rate, blood pressure, and breathing rate. The method also includes projecting a future trend of the plot, and, if the future trend varies from a desired future trend of the plot, determining a suggested modification of the patient's treatment plan to correct for the variation. Projecting a future trend can include using, for example, any one of a linear extrapolation technique, a conic extrapolation technique, and a polynomial extrapolation technique. In some embodiments, projecting a future trend includes extrapolating a sequence of future patient indicator data points following the patient indicator data points related to past treatment history of the patient. In some embodiments, the patient indicator data points related to past treatment history of the patient define a current trend, and projecting a future trend includes extrapolating a remedial trend to align the current trend with the desired future trend. The suggested corrective action can include adjusting an amount of fluid disposed within the restriction device, advising the patient to alter a level of physical activity, recommending that the patient adjust diet, and/or recommending that the patient adjust eating habits. In some embodiments, the method also includes displaying a notice of the suggested modification of the patient's treatment plan on a display device.

In other aspects, a system for affecting a weight loss treatment is provided. The system includes a data gathering device that can gather data that relates to a patient having an implanted restriction device that can form a restriction in the patient, with the data defining a current trend over time. The data gathering device can be implantable in or external to the patient. If external to the patient, the data gathering device in some embodiments includes a portable electronic unit configured to allow a user to input data that relates to the patient. The system also includes a processor that can be in electronic communication with the data gathering device and extrapolate a future trend from the current trend and, if the future trend deviates from a desired future trend, determine a suggested corrective action to address the deviation. The processor can be included in an external unit or implantable in the patient, e.g., an implantable sensor housing that houses the data gathering device and the processor and that can be in communication with the restriction device. In some embodiments, the system also includes an external display device that can display a notice of the suggested corrective action.

The following is a non-exhaustive list of embodiments of the invention that are or may be claimed.
1. A system for affecting a weight loss treatment, comprising: a data gathering device configured to gather data that relates to a patient having an implanted restriction device configured to form a restriction in the patient, the data defining a current trend over time; and
   a processor configured to be in electronic communication with the data gathering device and effective to extrapolate a future trend from the current trend and, if the future trend deviates from a desired future trend, to determine a suggested corrective action to address the deviation.
2. The system of embodiment 1, further comprising an external display device configured to display a notice of the suggested corrective action.
3. The system of embodiment 1, wherein the data gathering device is implantable in the patient.
4. The system of embodiment 1, wherein the data gathering device is external to the patient.
5. The system of embodiment 4, wherein the data gathering device includes a portable electronic unit configured to allow a user to input data that relates to the patient.
6. The system of embodiment 1, wherein the processor is implantable in the patient.
7. The system of embodiment 1, further comprising an implantable sensor housing that houses the data gathering device and the processor and that is configured to be in communication with the restriction device.
8. The system of embodiment 1, further comprising an external unit including the processor.
9. A method of affecting a weight loss treatment, comprising: extrapolating a sequence of future data values using at least two data values gathered in relation to a patient having an implanted restriction device configured to form a restriction in the patient; and if the sequence of future data values deviates from a desired sequence of future data values, determining a suggested corrective action to address the deviation.
10. The method of embodiment 9, wherein extrapolating a sequence of future data values includes extrapolating a sequence of future data values following the at least two data values.
11. The method of embodiment 9, wherein the at least two data values define a current trend, wherein the desired sequence of future data values define a desired trend, and wherein extrapolating a sequence of future data values includes extrapolating a remedial trend to align the current trend with the desired trend.
12. The method of embodiment 9, wherein the at least two data values reflect a patient condition including any one of weight, weight loss, weight gain, percent excess weight loss, body mass index, satiety level, body dimensions, heart rate, blood pressure, and breathing rate.
13. The method of embodiment 9, further comprising gathering the at least two data values using an implantable sensor device in communication with the restriction device.
14. The method of embodiment 9, further comprising gathering the at least two data values using a device external to the patient.
15. The method of embodiment 9, wherein the suggested corrective action includes modifying a treatment plan of the patient.
16. The method of embodiment 9, wherein the suggested corrective action includes any one of adjusting an amount of fluid disposed within the restriction device, advising the patient to alter a level of physical activity, recommending that the patient adjust diet, and recommending that the patient adjust eating habits.
17. The method of embodiment 9, further comprising displaying a notice of the suggested corrective action on a display device.
18. The method of embodiment 9, wherein extrapolating a sequence of future data values includes using any one of a linear extrapolation technique, a conic extrapolation technique, and a polynomial extrapolation technique.
19. A method of affecting a weight loss treatment, comprising: generating a plot showing a patient indicator plotted over time, wherein the plot includes patient indicator data points related to past treatment history of a patient having an implantable restriction device configured to form a restriction in the patient; projecting a future trend of the plot; and if the future trend varies from a desired future trend of the plot, determining a suggested modification of the patient's treatment plan to correct for the variation.
20. The method of embodiment 19, wherein projecting a future trend includes extrapolating a sequence of future patient indicator data points following the patient indicator data points related to past treatment history of the patient.
21. The method of embodiment 19, wherein the patient indicator data points related to past treatment history of the patient define a current trend, and wherein projecting a future trend includes extrapolating a remedial trend to align the current trend with the desired future trend.
22. The method of embodiment 19, wherein the patient indicator data points reflect a patient condition including any one of weight, weight loss, weight gain, percent excess weight loss, body mass index, satiety level, body dimension, heart rate, blood pressure, and breathing rate.
23. The method of embodiment 19, wherein the suggested modification includes any one of adjusting an amount of fluid disposed within the restriction device, advising the patient to alter a level of physical activity, recommending that the patient adjust diet, and recommending that the patient adjust eating habits.
24. The method of embodiment 19, wherein projecting a future trend includes using any one of a linear extrapolation technique, a conic extrapolation technique, and a polynomial extrapolation technique.
25. The method of embodiment 19, further comprising displaying a notice of the suggested modification of the patient's treatment plan on a display device.

### BRIEF DESCRIPTION OF THE DRAWINGS

The invention will be more fully understood from the following detailed description taken in conjunction with the accompanying drawings, in which:

FIG. 1A is a schematic diagram of an embodiment of a food intake restriction system;

FIG. 1B is a perspective view of an embodiment of an implantable portion of the food intake restriction system of FIG. 1A;

FIG. 2A is a perspective view of the food intake restriction device of FIG. 1A;

FIG. 2B is a schematic diagram of the food intake restriction device of FIG. 2A applied about the gastro-esophageal junction of a patient;

FIG. 3 is a perspective view of an embodiment of the injection port housing of FIG. 1A;

FIG. 4 is a perspective view of an embodiment of the sensor housing of FIG. 1A;

FIG. 5 illustrates an embodiment of the sensor housing of FIG. 1A;

FIG. 6 is a schematic of an embodiment of a variable resistance circuit for the pressure sensor of FIG. 5;

FIG. 7 is a block diagram showing an embodiment of internal and external components of the food intake restriction device of FIG. 1A;

FIG. 8 is a flow diagram showing an embodiment of a data analysis protocol for data measurements related to the food intake restriction system of FIG. 1A;

FIG. 9 is a perspective view of a display device;

FIG. 10 is a graphical representation of embodiments of data trend curves;

FIG. 11 is a schematic diagram of an embodiment of a data logger for recording data measurements related to the food intake restriction device of FIG. 1A;

FIG. 12 is a block diagram showing an embodiment of components of the data logger of FIG. 11;

FIG. 13 is a schematic diagram of an embodiment of a data logging system for recording data measurements related to the food intake restriction device of FIG. 1A;

FIG. 14 is a is a block diagram showing an embodiment of components of the data logging system of FIG. 13;

FIG. 15 is a perspective view of an embodiment of a gastric band system with a sensor positioned along a catheter;

FIG. 16 is a schematic view of an embodiment of a gastric band system with a sensor positioned within a catheter;

FIG. 17 is a perspective view of another embodiment of a gastric band system with a sensor positioned along a catheter; and

FIG. 18 is a schematic view of an embodiment of a gastric band system with a "T"-shaped sensor and catheter configuration.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention generally provides devices and methods for predicting performance of a restriction system for a patient. In general, the devices and methods can allow detection and prediction of a trajectory of a particular patient attribute, such as weight loss. Using previously gathered data values, data values defining a future outcome can be predicted and compared with a desired future outcome. If the future outcome deviates from the desired future outcome, one or more corrective actions can be suggested to a patient and/or a health care provider to help align the patient's treatment plan with the desired future outcome rather than the currently predicted future outcome. Such feed forward devices and methods can allow for early detection and quantification of divergence from a desired outcome. Early detection and quantification can help identify potential problems related to the patient's treatment before non-conformity gets too large, while small adjustments to a patient's treatment plan can be made, and before problems can discourage the patient or otherwise adversely affect efficacy of the restriction system and patient compliance.

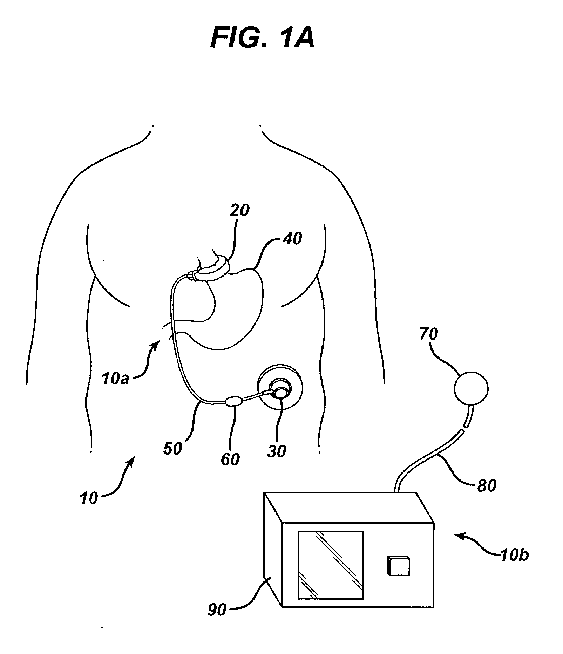

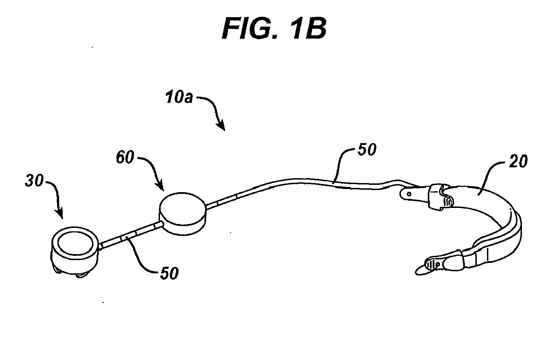

While the present invention can be used with a variety of restriction systems known in the art, FIG. 1A illustrates one exemplary embodiment of a food intake restriction system 10 in use in a patient. As shown, the system 10 generally includes an implantable portion 10a and an external portion 10b. FIG. 1B illustrates the implantable portion 10a outside of a patient. As shown, the implantable portion 10a includes an adjustable gastric band 20 that is configured to be positioned around the upper portion of a patient's stomach 40, and an injection port housing 30 that is fluidly coupled to the adjustable gastric band 20, e.g., via a catheter 50. The injection port 30 is adapted to allow fluid to be introduced into and removed from the gastric band 20 to thereby adjust the size of the band 20 and thus the pressure applied to the stomach 40. The injection port 30 can thus be implanted at a location within the body that is accessible through tissue. Typically, injection ports are positioned in the lateral subcostal region of the patient's abdomen under the skin and layers of fatty tissue. Surgeons also typically implant injection ports on the sternum of the patient.

The internal portion 10a can also include a sensing or measuring device that can be in fluid communication with the closed fluid circuit in the implantable portion 10a. In one embodiment, the sensing device is a pressure sensing device configured to measure the fluid pressure of the closed fluid circuit. While the measuring device can have various configurations and can be positioned anywhere along the internal portion 10a, including within the injection port 30 and as described further below, in the illustrated embodiment the measuring device is in the form of a pressure sensor that is disposed within a sensor housing 60 positioned adjacent to the injection port 30. The catheter 50 can include a first portion that is coupled between the gastric band 20 and the sensor housing 60 and a second portion that is coupled between the sensor housing 60 and the injection port 30. While it is understood that the sensing device can be configured to obtain data relating to one or more relevant parameters, generally it will be described herein in a context of a pressure sensing device.

In addition to sensing pressure of fluid within the internal portion 10a as described herein, pressure of fluid within the esophagus and/or the stomach 40 can also be sensed using any suitable device, such as an endoscopic manometer. By way of non-limiting example, such fluid pressure measurements can be compared against measured pressure of fluid within the internal portion 10a before, during, and/or after adjustment of pressure within the internal portion 10a. Other suitable uses for measured pressure within the esophagus and/or the stomach 40 will be appreciated by those skilled in the art.

As further shown in FIG. 1A, the external portion 10b generally includes a data reading device 70 that is configured to be positioned on the skin surface above the sensor housing 60 (which can be implanted beneath thick tissue, e.g., over 10 cm thick) to non-invasively communicate with the sensing housing 60 and thereby obtain pressure measurements. The data reading device 70 can optionally be electrically coupled (wirelessly or wired, as in this embodiment via an electrical cable assembly 80) to a control box 90 that can display the pressure measurements, other data obtained from the data reading device 70, and/or data alerts, as discussed further below. While shown in this example as located local to the patient, the control box 90 can be at a location local to or remote from the patient.

In some embodiments, the external portion 10b can include a sensing system configured to obtain data related to one or more relevant parameters, such as fluid pressure of the closed fluid circuit of the internal portion 10a. For example, pressure in the closed fluid circuit can be measured through a Huber needle in fluid communication with the injection port 30. An exemplary external pressure reading system is described in U.S. Publication No. 2006/0211912, entitled "External Pressure-Based Gastric Band Adjustment System and Method" which is hereby incorporated by reference.

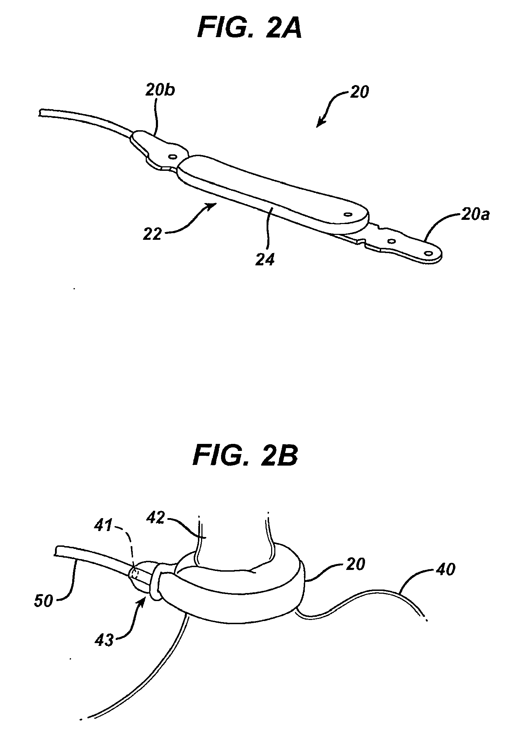

FIG. 2A shows the gastric band 20 in more detail. While the gastric band 20 can have a variety of configurations, and various gastric bands currently known in the art can be used with the present invention, in the illustrated embodiment the gastric band 20 has a generally elongate shape with a support structure 22 having first and second opposite ends 20a, 20b that can be formed in a loop such that the ends are secured to each other. Various mating techniques can be used to secure the ends 20a, 20b to one another. In the illustrated embodiment, the ends 20a, 20b are in the form of straps that mate together, with one laying on top of the other. In another embodiment, illustrated, for example, in FIGS. 1B and 2B, a support structure at one end of the gastric band 20 can include an opening through which the other end of the gastric band 20 can feed through to secure the ends to one another. The gastric band 20 can also include a variable volume member, such as an inflatable balloon 24, that is disposed or formed on one side of the support structure 22 and that is configured to be positioned adjacent to tissue. The balloon 24 can expand or contract against the outer wall of the stomach to form an adjustable stoma for controllably restricting food intake into the stomach.

A person skilled in the art will appreciate that the gastric band can have a variety of other configurations. Moreover, the various methods and devices disclosed herein have equal applicability to other types of implantable bands. For example, bands are used for the treatment of fecal incontinence, as described in U.S. Pat. No. 6,461,292 which is hereby incorporated by reference. Bands can also be used to treat urinary incontinence, as described in U.S. Publication No. 2003/0105385 which is hereby incorporated by reference. Bands can also be used to treat heartburn and/or acid reflux, as disclosed in U.S. Pat. No. 6,470,892 which is hereby incorporated by reference. Bands can also be used to treat impotence, as described in U.S. Publication No. 2003/0114729 which is hereby incorporated by reference.

FIG. 2B shows the adjustable gastric band 20 applied about the gastro-esophageal junction of a patient. As shown, the band 20 at least substantially encloses the upper portion of the stomach 40 near the junction with the patient's esophagus 42. After the band 20 is implanted, preferably in the deflated configuration wherein the band 20 contains little or no fluid, the band 20 can be inflated, e.g., using saline, to decrease the size of the stoma opening. A person skilled in the art will appreciate that various techniques, including mechanical and electrical techniques, can be used to adjust the band 20. FIG. 2B also shows an alternate location of a sensing device 41, disposed in a buckle 43 of the band 20.

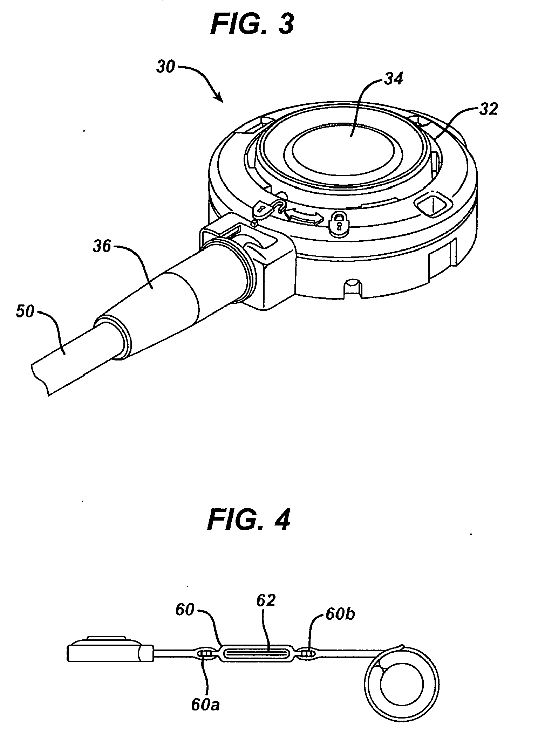

The fluid injection port 30 can also have a variety of configurations. In the embodiment shown in FIG. 3, the injection port 30 has a generally cylindrical housing with a distal or bottom surface and a perimeter wall extending proximally from the bottom surface and defining a proximal opening 32. The proximal opening 32 can include a needle-penetrable septum 34 extending there across and providing access to a fluid reservoir (not visible in FIG. 3) formed within the housing. The septum 34 is preferably placed in a proximal enough position such that the depth of the reservoir is sufficient enough to expose the open tip of a needle, such as a Huber needle, so that fluid transfer can take place. The septum 34 is preferably arranged so that it will self seal after being punctured by a needle and the needle is withdrawn. As further shown in FIG. 3, the port 30 can further include a catheter tube connection member 36 that is in fluid communication with the reservoir and that is configured to couple to a catheter (e.g., the catheter 50). A person skilled in the art will appreciate that the housing can be made from any number of materials, including stainless steel, titanium, or polymeric materials, and the septum 34 can likewise be made from any number of materials, including silicone.

The reading device 70 can also have a variety of configurations, and one exemplary pressure reading device is disclosed in more detail in commonly-owned U.S. Publication No. 2006/0189888 and U.S. Publication No. 2006/0199997, which are hereby incorporated by reference. In general, the reading device 70 can non-invasively measure the pressure of the fluid within the implanted portion 10a even when the pressure sensing device is implanted beneath thick (at least over 10 cm) subcutaneous fat tissue. The physician can hold the reading device 70 against the patient's skin near the location of the sensor housing 60, and/or other pressure sensing device location(s), and observe the pressure reading on a display on the control box 90. The data reading device 70 can also be removably attached to the patient, as discussed further below, such as during a prolonged examination, using straps, adhesives, and other well-known methods. The data reading device 70 can operate through conventional cloth or paper surgical drapes, and can also include a disposal cover (not shown) that may be replaced for each patient.

As indicated above, the system 10 can also include a pressure measuring device in communication with the closed fluid circuit and configured to measure pressure (e.g., fluid pressure) which corresponds to the amount of restriction applied by the adjustable gastric band 20 to the patient's stomach 40. Measuring the pressure can enable evaluation of the efficacy and functionality of the restriction created by a band adjustment. In the illustrated embodiment, as shown in FIG. 4, the pressure measuring device is in the form of a pressure sensor 62 disposed within the sensor housing 60. The pressure measuring device can, however, be disposed anywhere within the closed hydraulic circuit of the implantable portion, and various exemplary locations and configurations are disclosed in more detail in commonly-owned U.S. Publication No. 2006/0211913 entitled "Non-Invasive Pressure Measurement In a Fluid Adjustable Restrictive Device," filed on March 7, 2006 and hereby incorporated by reference. In general, the illustrated sensor housing 60 includes an inlet 60a and an outlet 60b that are in fluid communication with the fluid in the implantable portion 10a. An already-implanted catheter 50 can be retrofitted with the sensor housing 60, such as by severing the catheter 50 and inserting barbed connectors (or any other connectors, such as clamps, clips, adhesives, welding, etc.) into the severed ends of the catheter 50. The sensor 62 can be disposed within the housing 60 and be configured to respond to fluid pressure changes within the hydraulic circuit and convert the pressure changes into a usable form of data.

Various pressure sensors known in the art can be used as the pressure sensor 62, such as a wireless pressure sensor provided by CardioMEMS, Inc. of Atlanta, Georgia, though a suitable MEMS pressure sensor may be obtained from any other source, including but not limited to Integrated Sensing Systems, Inc. (ISSYS) of Ypsilanti, Michigan and Remon Medical Technologies, Inc. of Waltham, Massachusetts. One exemplary MEMS pressure sensor is described in U.S. Patent No. 6,855,115, the disclosure of which is incorporated by reference herein for illustrative purposes only. It will also be appreciated by a person skilled in the art that suitable pressure sensors can include, but are not limited to, capacitive, piezoresistive, silicon strain gauge, or ultrasonic (acoustic) pressure sensors, as well as various other devices capable of measuring pressure.

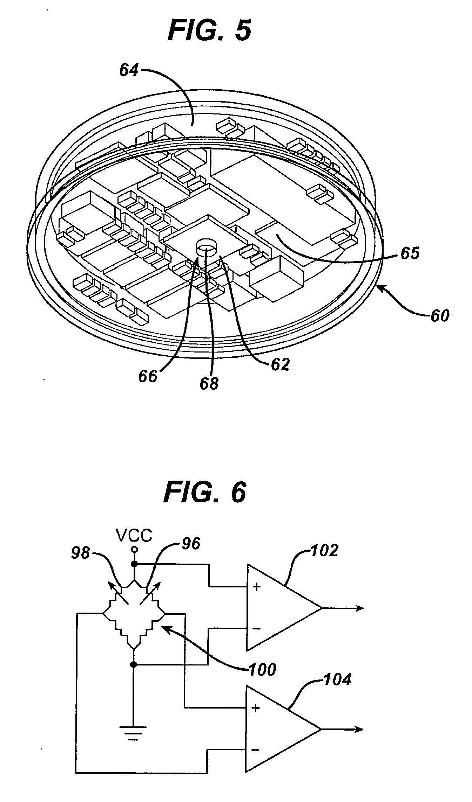

One embodiment of a configuration of the sensor housing 60 having the sensor 62 disposed within it is shown in FIG. 5. The sensor housing 60 in this example includes a motherboard that can serve as a hermetic container to prevent fluid from contacting any elements disposed within the sensor housing 60, except as discussed for the sensor 62. The sensor housing 60 can be made from any biocompatible material appropriate for use in a body, such as a ceramic, glass, polymer, biocompatible metal, and other similar types of material. Furthermore, the sensor housing 60 can be made from any one or more of transparent (as shown in FIG. 5), opaque, semi-opaque, and radio-opaque materials. A circuit board 64 including, among other elements, a microcontroller 65 (e.g., a processor), can also be disposed within the housing 60 to help process and communicate pressure measurements gathered by the sensor 62 and also other data gathered in relation to the system 10. As further discussed below, the circuit board 64 can also include a transcutaneous energy transfer (TET)/telemetry coil and a capacitor. Optionally, a temperature sensor can be integrated into the circuit board 64. The microcontroller 65, the TET/telemetry coil, the capacitor, and/or the temperature sensor can be in communication via the circuit board 64 or via any other suitable component(s). The TET/telemetry coil and capacitor can collectively form a tuned tank circuit for receiving power from the external portion 10b and transmitting measurements to a reading device, e.g., the reading device 70. Moreover, to the extent that a telemetry component associated with the sensor housing 60 is unable to reach a telemetry device external to the patient without some assistance, such assistance can be provided by any suitable number of relays (not shown) or other devices.

Fluid can enter the sensor housing 60 through an opening 66 located anywhere on the housing's surface (here, its bottom surface) and come into contact with a pressure sensing surface 68 of the sensor 62. The sensor 62 is typically hermetically sealed to the motherboard such that fluid entering the opening 66 cannot infiltrate and affect operation of the sensor 62 except at the pressure sensing surface 68. The sensor 62 can measure the pressure of fluid coming into contact with the pressure sensing surface 68 as fluid flows in and out of the opening 66. For example, the pressure sensing surface 68 can include a diaphragm having a deformable surface such that when fluid flows through the opening 66, the fluid impacts the surface of the diaphragm, causing the surface to mechanically displace. The mechanical displacement of the diaphragm can be converted to an electrical signal by a variable resistance circuit including a pair of variable resistance, silicon strain gauges. One strain gauge can be attached to a center portion of diaphragm to measure the displacement of the diaphragm, while the second, matched strain gauge can be attached near the outer edge of diaphragm. The strain gauges can be attached to the diaphragm with adhesives or can be diffused into the diaphragm structure. As fluid pressure within band 20 fluctuates, the surface of the diaphragm can deform up or down, thereby producing a resistance change in the center strain gauge.

One embodiment of a variable resistance circuit for the sensor 62 is shown in FIG. 6. The circuit includes first and second strain gauges 96, 98 that form the top two resistance elements of a half-compensated, Wheatstone bridge circuit 100. As the first strain gauge 96 reacts to the mechanical displacements of the sensor's diaphragm, the changing resistance of the first gauge 96 changes the potential across the top portion of the bridge circuit 100. The second strain gauge 98 is matched to the first strain gauge 96 and athermalizes the Wheatstone bridge circuit 100. First and second differential amplifiers 102, 104 are connected to the bridge circuit 100 to measure the change in potential within the bridge circuit 100 due to the variable resistance strain gauges 96, 98. In particular, the first differential amplifier 102 measures the voltage across the entire bridge circuit 100, while the second differential amplifier 104 measures the differential voltage across the strain gauge half of bridge circuit 100. The greater the differential between the strain gauge voltages, for a fixed voltage across the bridge, the greater the pressure difference. Output signals from the differential amplifiers 102, 104 can be applied to the microcontroller 65 integrated into the circuit board 64, and the microcontroller 65 can transmit the measured pressure data to a device external to the patient. If desired, a fully compensated Wheatstone bridge circuit can also be used to increase the sensitivity and accuracy of the pressure sensor 62. In a fully compensated bridge circuit, four strain gauges are attached to the surface of diaphragm rather than only two strain gauges.

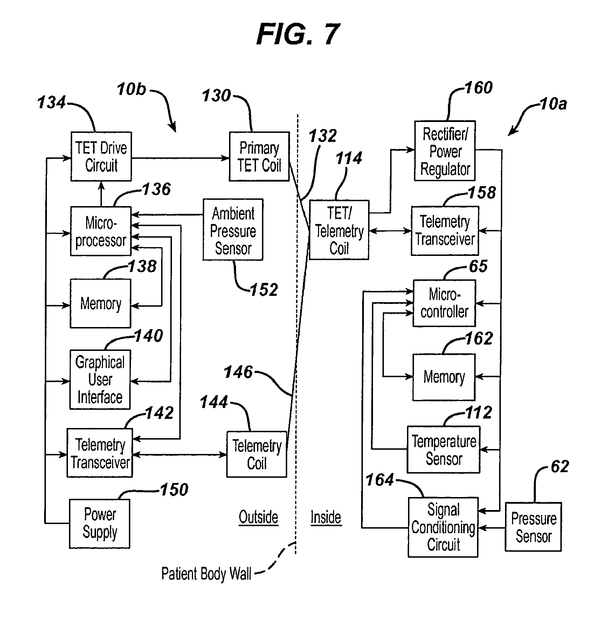

FIG. 7 illustrates one embodiment of components included in the internal and external portions 10a, 10b of the food intake restriction system 10. As shown in FIG. 7, the external portion 10b includes a primary TET coil 130 for transmitting a power signal 132 to the internal portion 10a. A telemetry coil 144 is also included for transmitting data signals to the internal portion 10a. The primary TET coil 130 and the telemetry coil 144 combine to form an antenna, e.g., the reading device 70. The external portion 10b, e.g., the control box 90, includes a TET drive circuit 134 for controlling the application of power to the primary TET coil 130. The TET drive circuit 134 is controlled by a microprocessor 136 having an associated memory 138. A graphical user interface 140 is connected to the microprocessor 136 for inputting patient information and displaying and/or printing data and physician instructions. Through the user interface 140, a user such as the patient or a clinician (e.g., a physician, a nurse, or any other medical personnel) can transmit an adjustment request to the physician and also enter reasons for the request. Additionally, the user interface 140 can enable the patient to read and respond to instructions from the physician and/or to alerts, as discussed further below.

The external portion 10b also includes a primary telemetry transceiver 142 for transmitting interrogation commands to and receiving data, including sensed data and any data related to a patient condition, from the implanted microcontroller 65. The primary transceiver 142 is electrically connected to the microprocessor 136 for inputting and receiving command and data signals. The primary transceiver 142 drives the telemetry coil 144 to resonate at a selected RF communication frequency. The resonating circuit can generate a downlink alternating magnetic field 146 that transmits command data to the microcontroller 65. Alternatively, the transceiver 142 can receive telemetry signals transmitted from a secondary TET/telemetry coil 114 in the internal portion 10a. The received data can be stored in the memory 138 associated with the microprocessor 136. A power supply 150 can supply energy to the control box 90 in order to power element(s) in the internal portion 10a. An ambient pressure sensor 152 is connected to microprocessor 136. The microprocessor 136 can use a signal from the ambient pressure sensor 152 to adjust the pressure measurements from the sensor 62 for variations in atmospheric pressure due to, for example, variations in barometric conditions or altitude, in order to increase the accuracy of pressure measurements.

FIG. 7 also illustrates components of the internal portion 10a, which in this embodiment are included in the sensor housing 60 (e.g., on the circuit board 64). As shown in FIG. 7, the secondary TET/telemetry coil 114 receives the power/communication signal 132 from the external antenna. The secondary coil 114 forms a tuned tank circuit that is inductively coupled with either the primary TET coil 130 to power the implant or the primary telemetry coil 144 to receive and transmit data. A telemetry transceiver 158 controls data exchange with the secondary coil 114. Additionally, the internal portion 10a includes a rectifier/power regulator 160, the microcontroller 65, a memory 162 associated with the microcontroller 65, a temperature sensor 112, the pressure sensor 62, and a signal conditioning circuit 164. The implanted components can transmit data stored in the memory 162, including pressure measurements (with or without adjustments due to temperature, etc.) from the sensor 62 and data related to a patient condition, to the control box 90 via the antenna (the primary TET coil 130 and the telemetry coil 144). Such transmitted data can be stored in the memory 138, adjusted for ambient pressure, shown on a display on the control box 90, and/or transmitted, possibly in real time, to a remote monitoring station at a location remote from the patient.

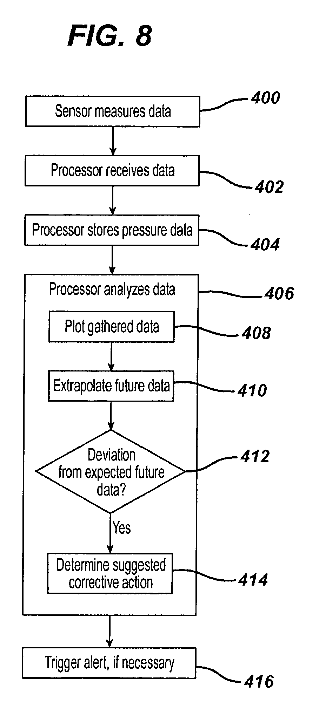

As illustrated in one embodiment of a process shown in FIG. 8, the sensor housing 60 can generally gather data, analyze the gathered data (e.g., using the microcontroller 65) to extrapolate future data based on the gathered data, determine if the extrapolated future data deviates from expected future data, and, if a deviation exists, determine at least one suggested corrective action to address the deviation. The sensor housing 60 can also provide an alert to the control box 90 (e.g., through the reading device 70) indicating the extrapolated data, existence of the deviation, and/or the suggested corrective action(s), which the control box 90 can provide to a user by, for example, displaying the alert (e.g., using the user interface 140). Such extrapolation and detection of a potential deviation can provide a patient, a physician, and/or any other user with evaluations of the efficacy of the band 20, including possible solutions to correct for any undesirable future patient condition(s), thereby allowing for improved functionality of the band 20, for timely (possibly in real time) attention to problems before they worsen or adversely affect patient morale, and/or for other diagnostic or treatment advantages. Alternatively, the sensor housing 60 can provide an alert to a smaller (typically wearable) indicator such as a wrist watch or pager-type device that can indicate to the patient that they need to allow the control box 90 to interrogate one or more elements included in the implanted portion 10a.

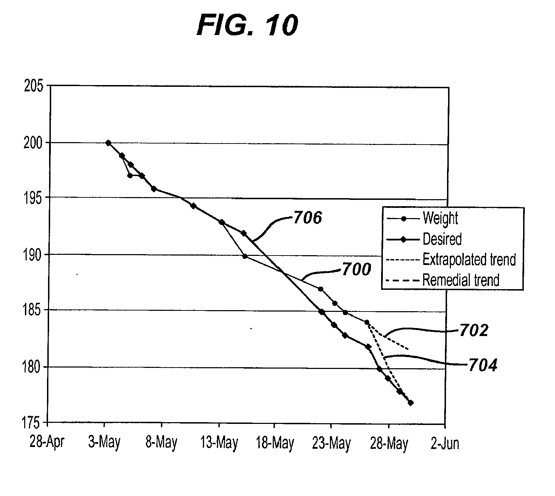

While the process shown in FIG. 8 is discussed with relation to the elements included in FIGS. 1A-7 and the plot shown in FIG. 10, a person skilled in the art will appreciate that the process can be modified to include more or fewer elements, reorganized or not, and can be performed in the system 10 or in another, similar system having other, similar elements. For example, the microcontroller 65 processes instructions in this embodiment, but any processor configured to process instructions for a system (e.g., a central processing unit, a microprocessor, a digital signal processing unit, application specific integrated circuits (ASICs), a state machine, an analog computer, an optical or photonic computer, logic circuitry, etc.) can be used. Furthermore, any processor at a location local to or remote from the patient, such as the microprocessor 136 in the external portion and a microprocessor 276 in a data logger 270 (described further below), can similarly process data.

Various types of data can be gathered for analysis by the microcontroller 65 either as sensed data (e.g., from the sensor 62 or any other sensing device) or as input data (e.g., transmitted from an external device to the microcontroller 65). Gathered data can also be received 402 by the microcontroller 65 in a variety of ways. The microcontroller 65 receives 402 at least one type of data for analysis, but the microcontroller 65 can receive 402 any number of different types of data in any combination.

One type of gathered data includes data measured 400 by a sensing device, which in this embodiment includes the sensor 62 measuring pressure but in other embodiments can include a sensing device measuring any type of data. In this embodiment, the sensor housing 60 can sense 400 a pressure of fluid disposed within the band 20 using the sensor 62. (The sensor 62 in this illustrated embodiment measures fluid pressure, but any sensed pressure data related to the band 20 can be handled as discussed herein.) The sensor 62 can transmit measured signals to the signal conditioning circuit 164, which can amplify the signals before the signal conditioning circuit 164 transmits the measured pressure data to the microcontroller 65. Alternatively, in some embodiments, the sensor 62 can directly transmit signals to the microcontroller 65. In this embodiment, the pressure sensor 62 provides pressure data at an update rate of approximately 20 Hz. Such a rate can provide a telemetry/TET mode cycle completion approximately every 50 ms. For example, the TET/telemetry coil 114 can provide TET for the sensor housing 60 for approximately 45 ms to power the sensor housing 60 and then provide telemetry of data for approximately 5 ms. Of course, any other switching topology can be used. It will also be appreciated that switching between TET and telemetry may be unnecessary. For example, the sensor housing 60 can be active, such that TET is not required. As another example, a second coil (not shown) can be added to the sensor housing 60, with one of the coils in the sensor housing 60 being dedicated to TET and the other to telemetry. Still other alternatives and variations will be apparent to those skilled in the art.

Another type of gathered data includes data related to a patient condition. Non-limiting examples of data related to a patient condition include weight, weight loss, weight gain, percent excess weight loss, body mass index (BMI), satiety level, body dimensions (e.g., waist, stomach, hips, thighs, arms, chest, etc.), heart rate (resting or breathing), blood pressure, breathing rate (resting or under exercise), and other similar types of data related to the patient and the patient's treatment with the restriction device 20. The microcontroller 65 can receive 402 patient condition data from a variety of sources, such as from a sensing device implanted in the patient (e.g., a sensing device disposed in the sensor housing 60, etc.) and from a device external to the patient (e.g., the control box 90 via the reading device 70, a data logger (discussed further below), etc.). If the microcontroller 65 receives 402 data from an external device, a sensing device may not measure 400 any data that the microcontroller 65 includes as part of its analysis (if such a sensing device is included in the internal portion 10a at all) because the microcontroller 65 can receive data to analyze from an external device.

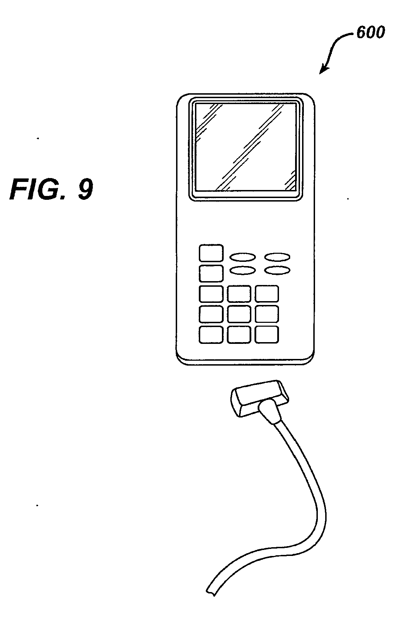

In some embodiments, the patient, a physician, and/or other user can enter data related to a patient condition into an external device such as a wired or wireless hand held display device 600, one embodiment of which is shown in FIG. 9, which can electronically communicate the input data to the microcontroller 65 over one or more wired and/or wireless communication links. A communication link can include any single or combination of two or more data transmission media including web-based systems utilizing high-speed cable or dial-up connections, public telephone lines, wireless RF networks, Bluetooth, ultrawideband (UWB), satellite, T1 lines or any other type of communication media suitable for transmitting data between remote locations. For example, a patient can enter his or her weight and the time and/or date into the display device 600 at a prescribed interval (e.g., daily, twice daily, weekly, etc.), and the entered weight, time, and/or date can be communicated to the microcontroller 65. For another example, the patient can enter information about his or her level of satiety one or more times a day (e.g., at regular intervals, a certain amount of time before or after a meal, etc.) into the display device 600. The patient can enter a number corresponding to a current level of satiety, e.g., based on a scale using one for hungry, three for satiated, five for content, seven for full, and nine for overstuffed. As another example, the patient can enter information about the types of food eaten at a certain time (e.g., a particular time or a time of day) into an input device, such the hand held device 600 or the user interface 140. The patient can enter a number corresponding to a particular food type (e.g., one for solid, two for liquid, etc.), select a food type from a provided list of specific foods or food types, take a picture of food to be eaten and upload it to the input device, etc. For still another example, the patient can step onto a scale which can electronically communicate the patient's weight to the microcontroller 65.

Having received 402 data, the microcontroller 65 can store 404 the data, e.g., in the memory 162. Any type of memory can be used for the memory 162, including but not limited to one or more of volatile (e.g., SRAM, etc.), non-volatile (e.g., flash, hard drive, etc.), or other memory. The microcontroller 65 can store any or all portions of gathered data in the memory 162. Although in this embodiment the microcontroller 65 stores gathered data before analyzing 406 the data as described below, the microcontroller 65 can store data in the memory 162 before and/or after analyzing the data, if the microcontroller 65 stores the data in the memory 162 at all (e.g., if the microcontroller 65 telemeters gathered data rather than storing it, if the microcontroller 65 selectively stores portions of raw data, etc.). Furthermore, the memory 162 can be used to store pre-selected information or pre-selected types of information. For example, the memory 162 can store maximum, minimum, and/or baseline measurements, fluoroscopic images or video of a patient swallowing, and/or any other information suitable for storing in the memory 162 as will be appreciated by those skilled in the art.

The microcontroller 65 can analyze 406 gathered data in a variety of ways. Typically, the microcontroller 65 analyzes a sequence of at least two data values measured over a period of time rather than analyzing every discrete measurement, thereby allowing for analysis of trends over time and saving processing resources by not necessarily having to continually analyze incoming data. In other words, the microcontroller 65 can store 404 gathered data in the memory 162 and retrieve and analyze any portion of the stored data every "X" minutes and/or upon signal from an external device. The microcontroller 65 can, however, evaluate individual data measurements (and/or a range of data), e.g., to identify invalid data and discard any invalid data.

Generally, in analyzing 406 data, the microcontroller 65 can follow a pre-programmed algorithm to predict a future trend considering the gathered data, determine if the future trend deviates from an expected trend, and, if a deviation exists, suggest a corrective action to address the deviation. One embodiment of such analysis is shown in FIG. 8.

The microcontroller 65 can plot 408 a curve including the gathered data such that the curve reflects a current trend of the data being plotted. A non-limiting example of a current trend curve 700 showing patient weight versus time is illustrated in FIG. 10. (The weights and times shown in FIG. 10 are examples only; the weights can include any values or ranges of values over any period of time. Furthermore, the current trend curve 700 plots weight, but the curve 700 can reflect any one or more gathered data types.) As mentioned above, the plot of gathered data can include any number of data values, although at least two data values are typically used to define a trend. The plotted gathered data values are typically sequential to allow for an accurate, continuous curve and are typically plotted versus time. Gathered data can be correlated to a time (hour, minute, day, a particular meal, etc.) by, for example, being time-stamped or being determined to be related to a particular meal based on one or more factors considered by the microcontroller 65, such as a combination of a time of day when a sensing device measured the data and a duration of pressure values above a zero or resting pressure level.

The microcontroller 65 can also extrapolate 410 future data using the gathered data. Typically, the microcontroller 65 uses one type of gathered data (e.g., weight, weight loss, etc.) in extrapolating 410 data, but the microcontroller 65 can correlate two or more types of gathered data in extrapolating 410 data (e.g., correlating heart rate and weight). The microcontroller 65 can use any one or more extrapolation techniques to extrapolate 410 future data. Non-limiting examples of extrapolation techniques include linear extrapolation (e.g., creating a tangent line beyond an end of the current trend curve 700 and extending the tangent line beyond the end of the current trend curve 700), conic extrapolation (e.g., creating a conic section using five data values near an end of the current trend curve 700), and polynomial extrapolation (e.g., creating a polynomial curve through all or a portion of the current trend curve 700 and extending the polynomial curve beyond an end of the current trend curve 700). Various software known in the art can be used to perform such extrapolation, such as Fityk (available under GNU General Public License), Ch (marketed by Softlntegration, Inc. of Davis, California), ZunZun.com (online curve fitting), and savetman.com (online curve fitting using least squares fit with weights).

Extrapolating 410 future data can include extrapolating a future trend curve 702 given the current trend curve 700, e.g., predicting data points to continue the current trend curve 700 beyond a time for which data has been gathered (or at least beyond a time which the microcontroller 65 is currently analyzing data). Alternatively or in addition, extrapolating 410 future data can include generating a remedial curve 704 to align the current trend curve 700 with a desired trend curve 706.

The desired trend curve 706 (e.g., data values that can be plotted by the microcontroller 65 to define the desired trend curve 706) is typically programmed into the microcontroller 65 by a physician based on at least one of an ideal goal for the patient (e.g., weights to be achieved over time), historical results of the patient (e.g., typical body mass index changes achieved by the patient over time), or, particularly for recently implanted bands, results for a typical patient or patients having similar profiles to the instant patient (e.g., typical breathing rates for patients having similar weight, age, exercise level, etc. as the instant patient). Desired trends can therefore vary between patients and even for an individual patient as the patient loses weight or otherwise experiences changes that can affect the patient's treatment plan. A desired trend is typically expressed as gathered data versus time, e.g., a curve that may or may not have a constant value over a particular time period. Moreover, the microcontroller 65 can generate the desired trend using previously gathered data, e.g., data stored in the memory 162.

The microcontroller 65 can also determine 412 if the future trend curve 702 deviates from the desired trend curve 706. If so, the microcontroller 65 can determine 414 at least one suggested corrective action to address the deviation, e.g., to help improve the chances of the patient achieving desired results. Suggested corrective actions generally include modifying the patient's treatment plan, which can involve internal and/or external adjustments, to help the patient's actual future results more closely follow the remedial curve 704 than the future trend curve 702 predicted given the current trend curve 700. A degree of corrective action can be indicated by a slope of the remedial curve 704 (e.g., eat "X" fewer calories per day). Generally, the larger the slope of the remedial curve 704, the more drastic the suggested corrective action(s), e.g., the more calories that should be suggested to the patient for daily consumption. Although, as mentioned above, the remedial curve 704 need not be extrapolated, and suggested corrective action(s) can generally address a deviation (e.g., eat more food if a current weight trend is below a desired weight trend) without considering a degree of corrective action. The microcontroller 65 can trigger 416 an alert to a physician, the patient, and/or to any number of other people indicating the deviation and/or the suggested corrective action(s). Alternatively, the data extrapolated from the current trend curve 700 by the microcontroller 65 can substantially equal the desired trend curve 706. Such a result indicates that the patient is substantially on track to achieve desired results and no alert need be triggered 416, although in some embodiments, an alert providing notice of a non-deviating trend can be provided.

The microcontroller 65 can trigger 416 an alert in a variety of ways. The microcontroller 65 can trigger an alert by, for example, communicating a signal to an external device (e.g., the control box 90, the display device 600, etc.) indicating the deviation and/or the suggested corrective action(s) and triggering notice of the alert. An alert can include any one or more of the following: an e-mail, a phone call, a text message, an audible signal, a mechanical vibration, a light or other visual display, a tactile display, a message displayed on an external device, an image displayed on an external device (e.g., a symbol indicating detection of a deviation, a projected body image based on the future trend curve 702, a morphing body image based on any combination of trend curves, etc.), or any other type of alert. Different alert patterns (e.g., varying audio signals, varying vibration patterns, etc.) can be used to signify different conditions. Two or more alerts can be provided to multiple people under similar conditions, although alerts may not be provided simultaneously to multiple people or be provided to anyone at all. The type of an alert can also vary relative to the magnitude of the deviation, the type of data being analyzed, and/or to the recipient of the alert. For example, with respect to alerts for physicians or other medical personnel, such alerts may be limited to those provided upon a deviation from the desired trend curve 706 above a certain threshold amount (e.g., a predicted weight at least a certain percentage above a desired amount on a certain date, a heart rate over a pre-programmed level, a remedial curve 704 slope above a pre-selected degree, etc.) that a physician may want to soon discuss with or evaluate in the patient. With respect to alerts for patients, such alerts may be limited to patient activity, such as those provided upon an indication that the patient is exercising too infrequently, eating too quickly, or consuming too few calories. A variety of other conditions under which alerts can be directed to a physician, a patient, and/or another person will be understood by those skilled in the art. Other suitable processes for detecting alert triggers, as well as ways in which the alerts can be provided, will be appreciated by those skilled in the art.

As mentioned above, gathered data (the data first analyzed by the microcontroller 65 or not) can be uploaded to an external unit such as the control box 90 (and/or other units located local or remote to the patient) to allow a person to physically evaluate and/or the control box 90 to electronically evaluate the patient's treatment and/or performance of elements included in the internal portion 10a over a designated time period. Also as mentioned above, in some embodiments, a processor included in the external portion 10b of the restriction system 10 (e.g., the microprocessor 136, the microprocessor 276, etc.) can receive 402, store 404, and/or analyze 406 gathered data. Such an external processor can also trigger 416 an alert, if necessary.

Data stored in the implantable memory 162 can be communicated to an external device in a variety of ways. In some embodiments, the microcontroller 65 continually communicates data (via the telemetry transceiver 158 and the secondary coil 114), and the data is only received when an appropriate receiving device, such as the antenna (the primary TET coil 130 and the telemetry coil 144), moves into sufficient proximity of it. In some embodiments, a download of data from the memory 162 can be triggered when an external device (e.g., the reading device 70) telemetrically provides power to the sensor housing, e.g., when the external device is moved in proximity of the sensor housing 60. The external device can be mobile (e.g., a wand or hand-held unit that can be waved or otherwise placed in proximity of the sensor housing 60) or stationary (e.g., a bedside, desk-mounted, or car-mounted box that the patient can move near). Telemetrically providing power to the sensor housing 60 can save power in the internal portion 10a because download communication power is supplied by the external portion 10b.

The external device can be configured to store data received from the sensor housing 60. The external device can be further configured to communicate the data to another external device, such as a base unit at a location remote from the patient. The external device (typically, the control box 90 or other device having a capability to display or otherwise provide an alert such as the hand held display device 600) can detect if the internal portion 10a communicated a signal indicating an alert and provide an alert as appropriate (e.g., displaying a warning notice, sending an e-mail message, etc.).

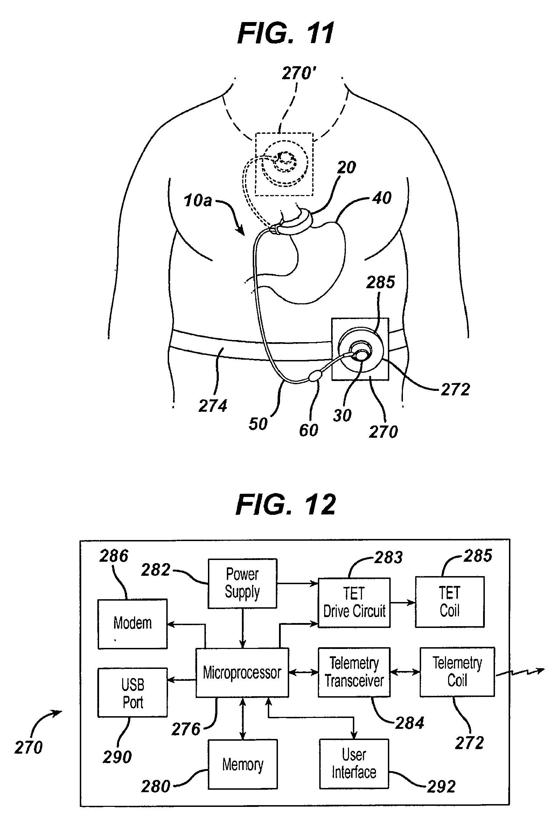

FIG. 11 illustrates an embodiment of an external device, a data logger 270, that can include a processor that can gather and analyze data over a period of time. The data logger 270 can function as a removably attached data reading device 70, mentioned above. In this example, the data logger 270 includes a wearable pack external to the patient worn on a belt 274 and positioned over or within communication range of the region under which the sensor housing 60 is implanted within the patient. Alternatively, the data logger 270 can be worn about the patient's neck, as shown by a device 270', such as when the injection port 30 is implanted on the patient's sternum and the port 30 includes a sensing device. In another embodiment, the data logger 270 is also implanted within the patient.

As shown in FIG. 11, the data logger 270 includes a TET coil 285 and a telemetry coil 272 which can be worn by the patient so as to lie adjacent to the internal portion 10a. The TET coil 285 can provide power to the implant, while the telemetry coil 272 can interrogate the implant and can receive data signals, including pressure measurements, through the secondary telemetry coil 114 in the implanted portion 10a. In another embodiment, the TET coil 285 and the telemetry coil 272 can be consolidated into a single coil and alternate between TET and telemetry functions at any suitable rate for any suitable durations.

The data logger 270 is typically worn during waking periods to record data during the patient's meals and daily routines. For example, pressure within the band 20 can be repeatedly sensed and transmitted to the data logger 270 at an update rate sufficient to measure peristaltic pulses against the band 20. Typically, this update rate is in the range of 10-20 pressure measurements per second, but any update range can be used. At the end of the day, or another set time period, the data logger 270 can be removed and recorded data can be downloaded to the external memory 138. The data can be uploaded from the memory 138 to a remote unit over one or more communication links during a subsequent communication session. Alternatively, data can be directly uploaded from the data logger 270 to a remote unit using one or more communication links. The data logger 270 can be configured to dock into another device, e.g., a docking station, that is configured to receive data communication from the data logger 270 and transmit the received data to a remote unit.

FIG. 12 shows the data logger 270 in greater detail. As shown in FIG. 12, the data logger 270 includes a microprocessor 276 for performing analysis as described above and/or for controlling telemetry communications with the internal portion 10a. The microprocessor 276 is connected to a memory 280 that can for example store pressure measurements from the internal portion 10a. In this embodiment, the memory 280 includes forty Mb of SRAM and is configured to store one hundred hours of time stamped pressure data, but any other type of storage can be used, and the memory 280 can store any amount of and any type of data. By way of non-limiting example, any other type of volatile memory or any type of non-volatile memory can be used. While the data logger 270 in this example is operational, measurements can be taken and stored in the memory 280 at a designated data rate controlled by the microprocessor 276.

The microprocessor 276 can be energized by a power supply 282. In one embodiment, the power supply 282 includes a rechargeable cell (not shown), such as a rechargeable battery. In some embodiments, the rechargeable cell is removable and can be recharged using a recharging unit and replaced with another rechargeable cell while the spent cell is recharging. In other embodiments, the rechargeable cell can be recharged by plugging a recharging adapter into the data logger 270 and a wall unit. In yet another embodiment, the rechargeable cell can be recharged wirelessly by a wireless recharging unit. In still another embodiment, the power supply 282 includes an ultra capacitor, which can also be recharged. Of course, any other type of power supply can be used.

To record data, the microprocessor 276 can initially transmit a power signal to the internal portion 10a via a TET drive circuit 283 and the TET coil 285. After transmitting the power signal, the microprocessor 276 can transmit an interrogation signal to the internal portion 10a via a telemetry transceiver 284 and the telemetry coil 272. The interrogation signal can be intercepted by the telemetry coil 114 and transmitted to the microcontroller 65. The microcontroller 65 can send responsive data, e.g., a heart rate measurement, an optionally-temperature-adjusted pressure reading from the sensor 62, etc., via the transceiver 158 and the secondary telemetry coil 114. The data can be received through the telemetry coil 272 and directed by the transceiver 284 to the microprocessor 276. The microprocessor 276 can store the data in its associated memory 280 and initiate the next interrogation request. If the microprocessor 65 can trigger an alert (in addition to or instead of the microprocessor 276 and/or any other processor), the microprocessor 276 can respond to an alert identified by the microcontroller 65, such as with a visual alert (e.g., flashing a light on the data logger 270, displaying a message on a user interface 292, etc.) and/or with an audible alert. The user interface 292 can include any number and types of features, including but not limited to a speaker, an LED, an LCD display, an on/off switch, etc. In some embodiments, the user interface 292 is configured to provide only output to the patient and does not permit the patient to provide input to the data logger 270. The user interface 292 thus includes an LED, which when lit shows that the power supply 282 is sufficiently charged and another, differently colored LED to show when the power supply 282 needs to be recharged, although such power indicators can be shown using any type and any combination of indicators such as one light that illuminates upon low power charge, an audible alert, an email alert, etc. In other embodiments, the user interface 292 can allow the patient to provide input to the data logger 270 and can accordingly include any suitable components and features.

When finished measuring and recording data, the data logger 270 can be removed from the patient and/or from the belt 274 and the recorded data downloaded to the control box 90 (and/or to any other external device). The data logger 270 can include a modem 286 for transmitting sensed pressure data directly to a remote base unit using a communication link. For example, the patient can connect the modem 286 to a telephone line (or other communication link), dial the physician's modem (if necessary), and select a "send" button on the user interface 292. Once connected, the microprocessor 276 can transmit stored data and/or data analysis through the phone line to a processor included in the remote unit. Alternatively, the data logger 270 can include a USB port 290 for connecting the logger 270 to the control box 90. The logger USB port 290 can be connected to a USB port included on the control box 90 and the "send" switch activated to download data to the memory 138 in the control box 90. After data is downloaded, the data logger 270 can be turned off through the user interface 292 or reset and placed back on the patient and/or the belt 274 for continued measurements.

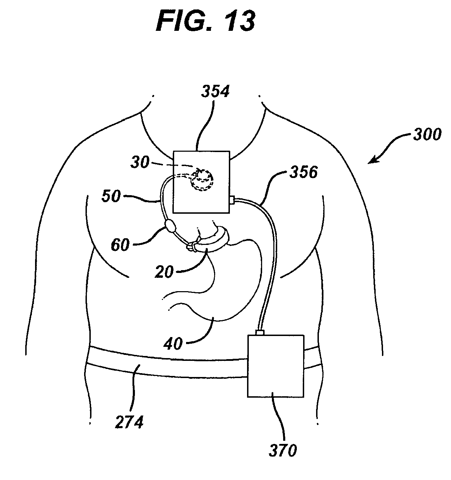

An alternate embodiment of a data logging system 300 is shown in FIG. 13. In this example, the data logging system 300 includes a coil head 354 and a data logger 370. The coil head 354 and the data logger 370 are in communication via a detachable cable 356. Any one or more suitable alternative communication links can be used in the place of the cable 356, including but not limited to a wireless transmitter/receiver system. In the illustrated embodiment, the coil head 354 is worn around the neck of the patient and is positioned generally over the injection port 30 and within communication range of the sensor housing 60. The data logger 370 is worn on the belt 274 about the patient's waist. Of course, these respective locations are merely exemplary, and either or both the coil head 354 and the data logger 370 can be positioned elsewhere. By way of non-limiting example, when the injection port 30 is implanted in the patient's abdomen, the coil head 354 can be worn on the belt 274. The coil head 354 and the data logger 370 are represented as simple blocks in FIG. 13 for illustrative purposes only, and either of the coil head 354 or the data logger 370 can be provided in a variety of shapes, sizes, and configurations.

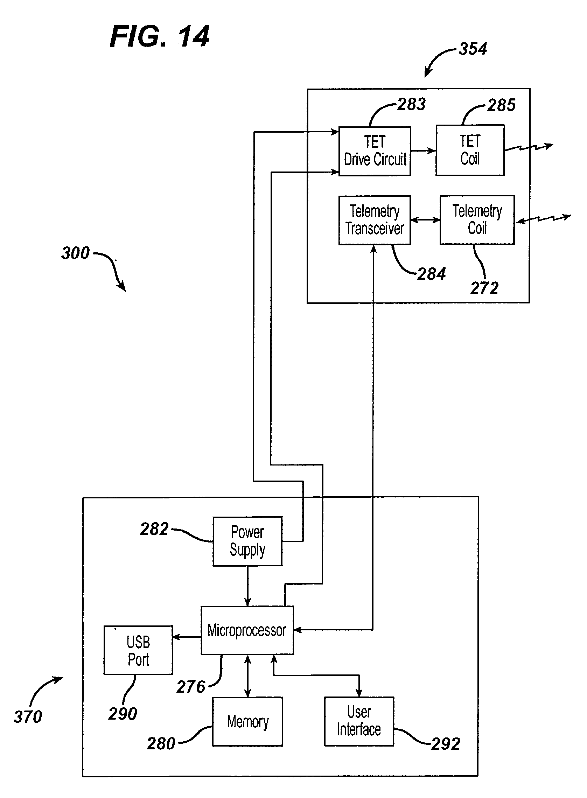

Exemplary components of the data logging system 300 are shown in FIG. 14. As shown, the data logger 370 includes the microprocessor 276, the memory 280, the power supply 282, the USB port 290, and the user interface 292. The coil head 354 includes the TET drive circuit 283, the telemetry transceiver 284, the TET coil 285, and the telemetry coil 272. The TET drive circuit 283 is configured to receive power from the power supply 282 via the cable 356. The TET drive circuit 283 is further configured to receive signals from the microprocessor 276 via the cable 356. The telemetry transceiver 284 is configured to receive signals from the microprocessor 276 and transmit signals to the microprocessor 276, via the cable 356. In another embodiment, the telemetry transceiver 284 is configured to only transmit signals to the microprocessor 276. The above discussion of such components with reference to FIG. 12 can also be applied to the components shown in FIG. 14. In the embodiment illustrated in FIG. 14, the coil head 354 and the data logger 370 can be viewed as a separation of components including the data logger 270 (described above) into two physically separate units. It will be appreciated by a person skilled in the art that any of the components shown in FIG. 14, as well as their relationships, functions, etc., can be varied in any suitable way.

In the present example, the coil head 354 is configured similar to and functions in a manner similar to the antenna (the primary TET coil 130 and the telemetry coil 144) described above. The TET coil 285 of coil head 354 is configured to provide power to the injection port 30. Of course, to the extent that any other devices (e.g., a pump, etc.) are implanted in the patient that are configured to receive power from the TET coil 285, the TET coil 285 can also provide power to such devices. Power provided by the TET coil 285 can be provided to the TET coil 285 by and regulated by the TET drive circuit 285, which can itself receive power from the power supply 282 via the cable 356. Such power provided to the TET drive circuit 283 can be regulated by the microprocessor 276 via the cable 356. In addition, or in the alternative, the microprocessor 276 can regulate the manner in which the TET drive circuit 285 provides power to the TET coil 285. While the present example contemplates the use of RF signaling through the TET coil 285, any other type of powering technique, as well as alternative power communicators, can be used. Other suitable configurations and relationships between these components, as well as alternative ways in which they may operate, will be appreciated by those skilled in the art.

The telemetry coil 272 of the coil head 354 is configured to receive signals from the coil 114, including signals indicative of the pressure within the implanted band system (e.g., pressure of fluid within the injection port 30, within the catheter 50, and/or within the adjustable band 20, pressure obtained using the pressure sensor 62, etc.) and signals indicative of temperature. The telemetry coil 272 can also receive any other type of signal representing any other type of information from any other source. Signals received by the telemetry coil 272 can be communicated to the telemetry transceiver 284, which can communicate such signals to the microprocessor 276 via the cable 356. The telemetry transceiver 284 can perform any appropriate translation or processing of signals received from the telemetry coil 272 before communicating signals to the microprocessor 276. Other suitable configurations and relationships between these components, as well as alternative ways in which they may operate, will be appreciated by those skilled in the art. It will also be appreciated that components may be combined. By way of non-limiting example, the TET coil 285 and the telemetry coil 272 can be consolidated into a single coil and alternate between TET and telemetry functions at any suitable rate for any suitable durations. In addition, while the present example contemplates the use of RF signaling through the telemetry coil 272, it will be appreciated that any other type of communication technique (e.g., ultrasonic, magnetic, etc.), as well as alternative communicators other than a coil, can be used.

In one exemplary use, the patient wears the coil head 354 and the data logger 370 throughout the day to record data in the memory 280. At night, the patient can decouple the data logger 370 from the coil head 354 and couple the data logger 370 with a docking station, e.g., the control box 90. While the data logger 370 and the control box 90 are coupled, the control box 90 can transmit data received from the data logger 370 to a remote unit. To the extent that the power supply 282 includes a rechargeable cell, the control box 90 can recharge the cell while the data logger 370 is coupled with the control box 90. However, a patient need not necessarily decouple the data logger 370 from the coil head 354 in order to couple the data logger 370 with the control box 90. Moreover, data can be recorded in the memory 280 and/or analyzed by the microprocessor 276 during the night in addition to or as an alternative to recording and/or analyzing such data during the day, and data can be recorded twenty-four hours a day. In that way, timing of data measuring, recordation, and analysis need not be limited to the daytime only.

As described above, the data logger 370 can receive, store, analyze, and communicate a variety of types of data relating the restriction system. By way of non-limiting example, the data logger 370 can receive, process, store, analyze, and/or communicate data relating to temperature, EKG measurements, eating frequency of the patient, the size of meals eaten by the patient, the amount of walking done by the patient, etc. It will therefore be appreciated by those skilled in the art that the data logger 370 can be configured to process received data to create additional data for communicating to the control box 90. For example, the data logger 370 can process pressure data obtained via the coil head 354 to create data indicative of the eating frequency of the patient. It will also be appreciated by those skilled in the art that the data logger 370 can include additional components to obtain non-pressure data. For example, the data logger 370 can include a pedometer or accelerometer (not shown) to obtain data relating to the amount of walking done by the patient. Data obtained by such additional components can be stored in the memory 280, communicated to the control box 90, and analyzed as discussed above. The data logger 370 can also include components for obtaining data to be factored in with other measurements, e.g., internal pressure measurements to account for effects of various conditions on the pressure. For example, the data logger 370 can include a barometer for measuring atmospheric pressure. In some embodiments, the data logger 370 includes an inclinometer or similar device to determine the angle at which the patient is oriented (e.g., standing, lying down, etc.), which can be factored into data to account for hydrostatic pressure effects caused by a patient's orientation. Alternatively, an inclinometer or other device for obtaining non-pressure data can be physically separate from the data logger 370 (e.g., implanted). Still other types of data, ways in which such data may be obtained, and ways in which such data may be used will be appreciated by those skilled in the art.

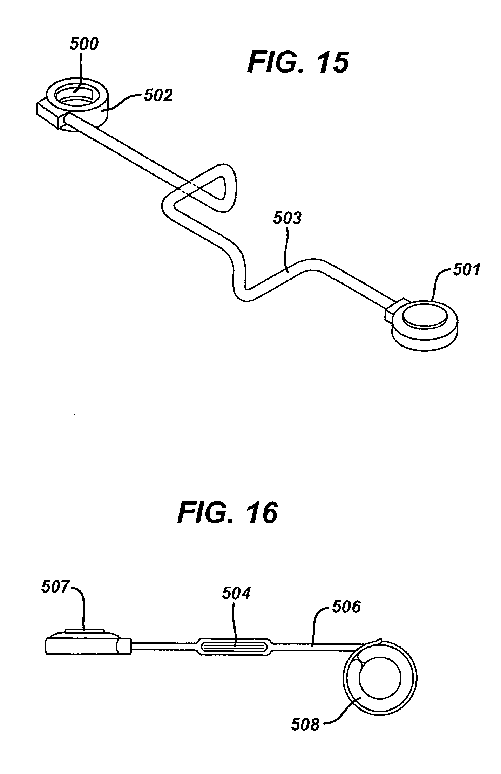

While embodiments described above include the use of a sensing device within the sensor housing 60 removably joined to the catheter 50, a sensing device can be located elsewhere within a patient. For example, a sensing device could be included in the port housing 30. In another embodiment, shown in FIG. 15, a sensing device 500 can be located within a gastric band 502, such as in an inflatable portion of gastric band 502. To the extent that the gastric band 502 includes a resilient portion and a non-resilient portion, the sensing device 500 can be secured to either or neither of the resilient portion or non-resilient portion. In any case, the sensing device 500 can, for example, sense and communicate fluid pressure within the gastric band 502 before, during, and after fluid is added to or withdrawn from gastric band 502 via an injection port 501 and a catheter 503. The sensing device 500 can be used when a pump (not shown) or any other device is used to adjust pressure within the gastric band 502.

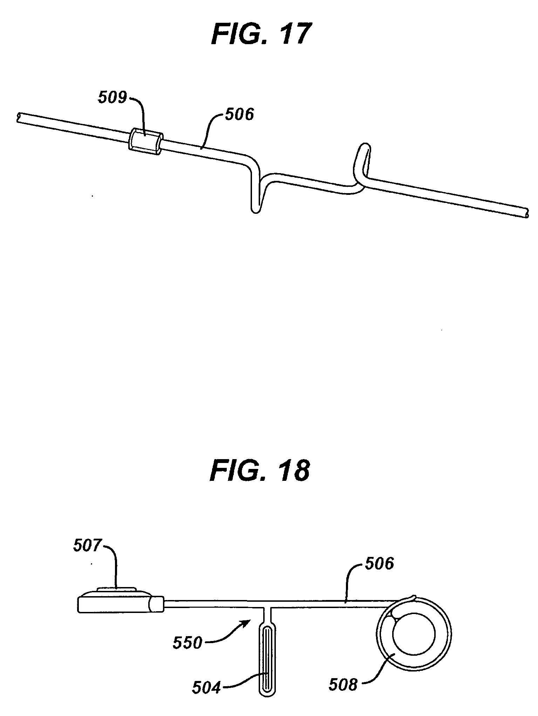

Alternatively, as shown in FIG. 16, a sensing device 504 can be located within a catheter 506 positioned between a gastric band 508 and a port 507, pump, reservoir, or other device in fluid communication with the catheter 506. As another variation, an example of which is shown in FIG. 17, a sensing device 509 can be fixedly secured in-line with a catheter 506, while not residing within catheter 506.

Yet another variation is shown in FIG. 18, which illustrates a catheter 506 having a "T"-shaped intersection 550. A sensing device 504 is disposed in the arm of the "T"-shaped intersection 550 that is perpendicular to the catheter 506 and is in fluid communication with the catheter 506. In one embodiment, the "T"-shaped intersection 550 is integrally formed with the catheter 506 (as shown). In another embodiment, the "T"-shaped intersection 550 is a separate component joined to the catheter 506 (e.g., using barbed connectors, etc.). Other suitable ways in which the "T"-shaped intersection 550 can be provided will be appreciated by those skilled in the art. Similarly, other ways in which a sensing device 504 can be provided within, in-line with, or adjacent to the catheter 506 will be appreciated by those skilled in the art.

In yet another embodiment (not depicted), a sensing device can be located at the interface of an injection port and a catheter, and/or at the interface of a gastric band and a catheter. Still other suitable locations for a sensing device will be appreciated by those skilled in the art, including but not limited to any location in or adjacent to the fluid path of a gastric band system. In addition, a sensing device can be positioned within (e.g., against an inner wall of) a gastric band, a catheter, and a buckle, or alternatively, a portion of such band, catheter, and buckle can include a protrusion extending outwardly therefrom to house at least a portion of the corresponding sensing device. Other suitable configurations for housing a sensing device within or adjacent to a band, catheter, or buckle will be appreciated by those skilled in the art.

In another embodiment, a plurality of sensing devices can be used. For example, a gastric band system can include a pressure sensor within a gastric band in addition to a pressure sensor within a catheter that is in fluid communication with the gastric band. Such a plurality of pressure sensors can provide an indication of how well fluid pressure is distributed among components of a gastric band system. Such a plurality of pressure sensors can also provide greater accuracy in pressure readings, reduce the likelihood of catheter obstruction (e.g., pinching) affecting pressure reading, reduce effects of hydrostatic pressure changes from patient movement, and/or provide one or more other results. Any system that includes a plurality of pressure sensors can include a pressure sensor in a port housing and/or a pressure sensor external to the patient (e.g., a pressure sensor in a syringe or in a pressure sensor portion coupled with a syringe), in addition to any of the implanted pressure sensors described above. Furthermore, a device such as an internal or external inclinometer (or a substitute therefor) may be used to determine the angle at which the patient and/or the internal portion is oriented (e.g., standing, lying down, etc.), which may be factored into pressure data sensed by one or more sensors to account for hydrostatic pressure effects caused by a patient's orientation. Such a factor (or any other factor) may be accounted for prior to or in conjunction with the rendering of a pressure reading.

A person skilled in the art will appreciate that the present invention has application in conventional endoscopic and open surgical instrumentation as well application in robotic-assisted surgery.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A system for affecting a weight loss treatment, comprising:
a data gathering device configured to gather data that relates to a patient having an implanted restriction device configured to form a restriction in the patient, the data defining a current trend over time; and
a processor configured to be in electronic communication with the data gathering device and effective to extrapolate a future trend from the current trend and, if the future trend deviates from a desired future trend, to determine a suggested corrective action to address the deviation.

2. The system of claim 1, further comprising an external display device configured to display a notice of the suggested corrective action.

3. The system of claim 1, wherein the data gathering device is implantable in the patient.

4. The system of claim 1, wherein the data gathering device is external to the patient.

5. The system of claim 4, wherein the data gathering device includes a portable electronic unit configured to allow a user to input data that relates to the patient.

6. The system of claim 1, wherein the processor is implantable in the patient.

7. The system of claim 1, further comprising an implantable sensor housing that houses the data gathering device and the processor and that is configured to be in communication with the restriction device.

8. The system of claim 1, further comprising an external unit including the processor.

9. A method of affecting a weight loss treatment, comprising:
extrapolating a sequence of future data values using at least two data values gathered in relation to a patient having an implanted restriction device configured to form a restriction in the patient; and
if the sequence of future data values deviates from a desired sequence of future data values, determining a suggested corrective action to address the deviation.

10. The method of claim 9, wherein extrapolating a sequence of future data values includes extrapolating a sequence of future data values following the at least two data values.

11. The method of claim 9, wherein the at least two data values define a current trend, wherein the desired sequence of future data values define a desired trend, and wherein extrapolating a sequence of future data values includes extrapolating a remedial trend to align the current trend with the desired trend.

12. The method of claim 9, wherein the at least two data values reflect a patient condition including any one of weight, weight loss, weight gain, percent excess weight loss, body mass index, satiety level, body dimensions, heart rate, blood pressure, and breathing rate.

13. The method of claim 9, further comprising gathering the at least two data values using an implantable sensor device in communication with the restriction device.

14. The method of claim 9, further comprising gathering the at least two data values using a device external to the patient.

15. A method of affecting a weight loss treatment, comprising:
generating a plot showing a patient indicator plotted over time, wherein the plot includes patient indicator data points related to past treatment history of a patient having an implantable restriction device configured to form a restriction in the patient;
projecting a future trend of the plot; and
if the future trend varies from a desired future trend of the plot, determining a suggested modification of the patient's treatment plan to correct for the variation.
